# EUROPÄISCHE PATENTSCHRIFT

(11) **EP 3 886 753 B1**
(45) Veröffentlichungstag und Bekanntmachung des Hinweises auf die Patenterteilung: **02.03.2022**
(21) Anmeldenummer: 20820355.4
(22) Anmeldetag: 02.12.2020
(51) Int. Cl.: A61B 50/30, A61F 2/00, A61B 50/00

(54) **VERBUNDVERPACKUNG ZUR ERLEICHTERTEN ASEPTISCHEN PRÄSENTATION VON MEDIZINPRODUKTEN**
COMPOSITE PACKAGING FACILITATING AN ASEPTIC PRESENTATION OF MEDICINAL PRODUCTS
EMBALLAGE COMPOSITE FACILITANT LA PRÉSENTATION ASEPTIQUE DE PRODUITS MÉDICINAUX

(30) Priorität: 04.12.2019 DE 102019133076
(43) Veröffentlichungstag der Anmeldung: 06.10.2021
(73) Patentinhaber: Aesculap AG, 78532 Tuttlingen (DE)
(72) Erfinder: HENKE, Matthias, 78048 Villingen-Schwenningen (DE)
(74) Vertreter: Winter, Brandl - Partnerschaft mbB
(86) Internationale Anmeldenummer: PCT/EP2020/084340
(87) Internationale Veröffentlichungsnummer: WO 2021/110783

(56) Entgegenhaltungen:
- EP-A1- 3 357 444
- DE-A1- 19 603 476
- US-A1- 2002 098 139
- US-A1- 2019 274 809

## Beschreibung

### Technisches Gebiet

Die vorliegende Offenbarung betrifft eine Sterilverpackung mit vorzugsweise einer inneren ersten Sterilbarriere und einer (obligatorischen) äußeren, zur ggf. vorhandenen ersten Sterilbarriere getrennt und unabhängig ausgebildeten zweiten Sterilbarriere für eine adäquate Verpackung von sterilen Medizinprodukten, insbesondere von Implantaten.

### Hintergrund der Erfindung

Medizinprodukte, wie beispielsweise Implantate, müssen in einer entsprechenden Verpackung, die eine Kontamination durch Mikroorganismen verhindert, bis zu ihrer Verwendung untergebracht werden. Dabei gelten für Verpackungen von Medizinprodukten bzw. Sterilgut im Allgemeinen bestimmte offizielle Normen wie die DIN EN ISO 11607 Teil 1 und Teil 2 und die EN 868 2-10, deren Anforderungen Hersteller von Medizinprodukten und auch Einrichtungen des Gesundheitswesens, wie z.B. Krankenhäuser, gerecht werden müssen, um eine adäquate und zeitgleich sichere und an das jeweilige Medizinprodukt angepasste Verpackung zu gewährleisten. So beschreibt die DIN EN ISO 11607 Teil 1 zum Beispiel die allgemeinen Anforderungen an Materialien, Sterilbarrieresysteme und Verpackungssysteme.

Bei der Auswahl der richtigen Verpackung spielen mehrere Aspekte wie beispielsweise die Beschaffenheit des zu verpackenden Medizinproduktes, die Anforderungen der Anwender, strukturelle Gegebenheiten und die Transportlogistik eine entscheidende Rolle. Jedoch sind auch eine entsprechende Anwenderfreundlichkeit und Sicherheitsaspekte von hoher Bedeutung. So muss eine Verpackung zum Beispiel ein einfaches Befüllen mit dem Produkt und ein keimdichtes Verschließen gewährleisten, und gleichzeitig so robust sein, dass sie Beschädigungen verhindert. Auch eine angemessene Kennzeichnung der Verpackung bzw. des Inhalts und die Sterilisation der Verpackung muss möglich sein. Da der Verlust der Sterilität eines Medizinproduktes weniger von der Lagerdauer abhängt, sondern von äußeren Einflüssen und Einwirkungen während des Transports und der Lagerung sollte die Verpackung eines Medizinproduktes auch in Hinblick auf die Transport- und Lagerbedingungen des jeweiligen Medizinproduktes ausgewählt werden. So kann beispielsweise ein vielfaches Handhaben von Klarsichtverpackungen zu einem Brechen des Materials, zu einem Lösen der Siegelnaht oder zur Bildung von Kanälen führen, oder Perforationen bedingen.

Implantate wie zum Beispiel Knie- oder Hüftimplantate werden heute für gewöhnlich doppelt steril-verpackt. Das heißt, das jeweilige Implantat ist in zwei aufeinanderfolgenden Blistern, Peelbeuteln, einer Kombination von beidem, oder ähnlichem verpackt, um eine aseptische/keimfreie Präsentation im Operationssaal zu ermöglichen. In der Praxis bedarf es für eine korrekte aseptische Präsentation doppelt steril-verpackter Medizinprodukte während einer Operation neben dem Arzt, der das Medizinprodukt letztendlich in den Patienten einsetzt oder im Verlauf der Operation anwendet, zwei OP-Schwestern. Dabei übernimmt eine der beiden OP-Schwestern die "unsterile Rolle", d.h. das Öffnen einer vorhandenen Umverpackung, in der das doppelt steril-verpackte Medizinprodukt untergebracht ist und das Öffnen des äußeren als zweite Sterilbarriere ausgebildeten Blisters, Peelbeutels o.ä., während die andere der beiden OP-Schwestern die "sterile Rolle" übernimmt und für ein korrektes Öffnen des inneren als erste Sterilbarriere ausgebildeten Blisters, Peelbeutels o.ä. verantwortlich ist.

Es ist aber bei bestimmten medizinischen Produkten auch eine einfache Sterilverpackung ausreichend, wie beispielsweise bei Gewebeklebern, aber auch Einwegskalpellen oder einigen Produkten in der Wirbelsäulenchirurgie.

Fast im gesamten Bereich der Lebensmittelverpackung werden mittlerweile sogenannte Verbundverpackungen verwendet. Verbundverpackungen bestehen aus Verbundwerkstoffen/Verbundmaterialien, d.h. sie bestehen aus zwei oder mehreren miteinander verbundenen Materialien, die andere Werkstoffeigenschaften besitzen als die einzelnen Materialkomponenten und die Eigenschaften der einzelnen Materialkomponenten sogar übertreffen. Da ein einzelner Werkstoff nicht alle gewünschten Eigenschaften bieten kann, kombiniert man unterschiedliche Materialien zu einem Verbund, der die gewünschten Eigenschaften aufweist und gleichzeitig den Anforderungen an Verpackungen in der Lebensmittelindustrie entspricht.

Das bekannteste Beispiel für Verbundverpackungen in der Lebensmittelindustrie sind Getränkekartons bzw. Einwegverpackungen für Getränke oder flüssige Nahrungsmittel. Sie bestehen aus Karton und Kunststoff, sowie häufig einer dünnen Schicht Aluminium, die vollflächig miteinander verbunden und von Hand nicht voneinander trennbar sind. Ein derartiger Aufbau schützt vor Licht und Sauerstoff und unterdrückt bzw. verlangsamt auf diese Weise Oxidationsprozesse und die Zerstörung von Vitaminen und Aromen der verpackten Lebensmittel außerhalb der Kühlkette.

In Anbetracht der umfangreichen Vorteile, die Verbundverpackungen je nach Zusammensetzung bieten können, erscheint die Verwendung von Verbundverpackungen bzw. Verbundmaterialien auch bei der Verpackung von sterilen Medizinprodukten, und insbesondere von Implantaten, sinnvoll zu sein, um den zahlreichen Anforderungen, die an Verpackungen von Medizinprodukten gestellt werden, auf effiziente Weise gerecht zu werden.

### Stand der Technik

Eine aktuell geläufige Verpackungslösung aus dem Stand der Technik sind Doppel-Sterilbarriere-Verpackungen von Medizinprodukten, welche beispielsweise aus der EP 3 357 444 A1 sowie der DE 196 03 476 A1 bekannt sind. Derartige Sterilverpackungen kommen bei einem Großteil von Medizinprodukteherstellern zum Einsatz und bestehen aus einer inneren ersten Sterilbarriere und einer äußeren zweiten Sterilbarriere, wobei für die Ausbildung/Erzeugung beider Sterilbarrieren Blister, Peelbeutel o.ä. verwendet werden. Das heißt, ein zu verpackendes steriles Medizinprodukt wird in einem inneren als erste Sterilbarriere ausgebildeten Blister, Peelbeutel o.ä. untergebracht. Dieser innere Blister, Peelbeutel o.ä. wird wiederum in einen äußeren als zweite Sterilbarriere ausgebildeten Blister, Peelbeutel o.ä. eingeführt. In der US 2019 / 274 809 A1 wird dieser äußere Blister, Peelbeutel o.ä., der den inneren Blister, Peelbeutel o.ä. und das sich darin befindliche sterile Medizinprodukt enthält, im Anschluss in einen Umkarton, welcher für einen besseren Schutz während des Transports ggf. zusätzlich mit einer Polsterung versehen sein kann, untergebracht. Der Umkarton kann zum Schutz schließlich ggf. zusätzlich von Zellophan umgeben sein. Des Weiteren ist eine Aufbewahrungs- / Transportverpackung für Medizinprodukte aus dem vorstehend erwähnten Verbundmaterial, beispielsweise aus Karton und einer laminierten Schicht, aus der US 2002 / 098 139 A1 bekannt.

Um ein derart doppelt steril-verpacktes Medizinprodukt entsprechend aseptisch während einer Operation zu präsentieren, bedarf es wie bereits vorstehend angesprochen sowohl eine "unsterile OP-Schwester", als auch eine "sterile OP-Schwester". Die "unsterile OP-Schwester" entfernt während der Operation in einem unsterilen Bereich zuallererst ggf. das den Umkarton umgebende Zellophan und öffnet den Umkarton bzw. klappt diesen auf, wodurch das doppelt steril-verpackte Medizinprodukt präsentiert wird. Im Anschluss daran nimmt die "unsterile OP-Schwester" das doppelt steril-verpackte Medizinprodukt oder genauer gesagt den äußeren als zweite Sterilbarriere ausgebildeten Blister, Peelbeutel o.ä., in welchem sich der innere als erste Sterilbarriere ausgebildete Blister, Peelbeutel o.ä. und das darin untergebrachte sterile Medizinprodukt befinden, aus dem Umkarton. Nun wird der äußere als zweite Sterilbarriere ausgebildete Blister, Peelbeutel o.ä. von der "unsterilen OP-Schwester" aseptisch geöffnet und der innere als erste Sterilbarriere ausgebildete Blister, Peelbeutel o.ä. der "sterilen OP-Schwester", die sich in einem sterilen Bereich befindet, angereicht/präsentiert. Der innere als erste Sterilbarriere ausgebildete Blister, Peelbeutel o.ä. inklusive dem darin enthaltenen sterilen Medizinprodukt wird dann von der "sterilen OP-Schwester" aus dem geöffneten äußeren als zweite Sterilbarriere ausgebildeten Blister, Peelbeutel o.ä. entnommen und im Sterilbereich des Operationssaals geöffnet. Das sich darin befindende sterile Medizinprodukt wird schließlich dem operierenden Arzt angereicht und am und/oder im Patienten angewendet.

Dabei muss die "unsterile OP-Schwester" beim Öffnen des äußeren als zweite Sterilbarriere ausgebildeten Blisters, Peelbeutels o.ä. darauf achten den inneren als erste Sterilbarriere ausgebildeten Blister, Peelbeutel o.ä. nicht durch inadäquates Handling unsteril zu machen. Darüber hinaus müssen sowohl die "unsterile Schwester", als auch die "sterile Schwester" während allen vorstehend beschriebenen Schritten genau darauf achten, dass das Medizinprodukt seine Sterilität beibehält und weder auf den Boden fällt, noch anderweitig beschädigt wird, da ein unsteriles und/oder beschädigtes Medizinprodukt nicht mehr zum Einsatz an einem Patienten kommen kann/darf. Des Weiteren sind die gebräuchlichen Verpackungen, wie zum Beispiel Peelbeutel, fest versiegelt, weshalb sie sich häufig nur sehr schwer oder ruckartig öffnen lassen. Für das Öffnen müssen die OP-Schwestern daher verhältnismäßig viel Kraft aufbringen. Diese Umstände und der oftmals vorhandene Zeitdruck während Operationen führen dazu, dass es oft zu Fehlern bei der aseptischen Präsentation von Medizinprodukten bzw. zu einem ungewünschten Verkomplizieren der aseptischen Präsentation von Medizinprodukten kommt.

Doppelt steril-verpackte Medizinprodukte gemäß dem Stand der Technik haben ferner einen recht großen Gesamtverpackungsaufwand und nehmen vergleichsweise viel Verpackungsvolumen in Anspruch.

Aber auch im Fall einer einzigen Sterilbarriere wird das darin steril-verpackte medizinische Produkt in einer zusätzlichen Umverteilung vorzugsweise aus Karton eingepackt was den Verpackungsaufwand insgesamt erhöht.

Daher gibt es Bedarf an Einzel- und Doppel-Sterilbarriere-Verpackungen für sterile Medizinprodukte, insbesondere für Implantate, die eine einfachere und unkompliziertere aseptische Präsentation von sterilen Medizinprodukten während einer Operation ermöglichen, und dabei zeitgleich deutlich weniger Verpackungsvolumen beanspruchen und weniger Verpackungsmüll erzeugen als vergleichbare Sterilverpackungen aus dem Stand der Technik.

### Allgemeine Beschreibung der Erfindung

Angesichts der vorstehend erläuterten Problematik besteht daher eine grundsätzliche Aufgabe der vorliegenden Erfindung darin, eine Sterilverpackung mit Doppel-Sterilbarriere für sterile Medizinprodukte, insbesondere Implantate, bereitzustellen, die trotz zusätzlicher Umverpackung insgesamt Verpackungsmaterial und/oder Verpackungsaufwand einspart und damit Verpackungsmüll einerseits und für den Hersteller anfallende Kosten für die Verpackung von Medizinprodukten sowie den Verpackungsvorgang andererseits deutlich reduziert.

In diesem Zusammenhang besteht eine weitere Aufgabe der vorliegenden Erfindung darin die aseptische Präsentation verpackter steriler Medizinprodukte mit Hilfe eines praktischeren Öffnungsmechanismus zu vereinfachen und ein umständliches Entpacken des Medizinproduktes zu vermeiden. Zeitgleich können durch die vorliegende Erfindung die einzelnen Entpackungsschritte der "unsterilen OP-Schwester" reduziert werden, was den Arbeitsaufwand minimiert und zu einer Zeitersparnis während einer Operation führt. Darüber hinaus wird durch die Reduktion der Entpackungsschritte und dem vereinfachten Öffnungsmechanismus auch das Risiko gesenkt, dass das Medizinprodukt während des Entpackungsprozesses herunterfällt, beschädigt oder allgemein unsteril gemacht wird.

Diese Aufgabe wird durch eine Sterilverpackung mit den Merkmalen des Patentanspruchs 1 gelöst. Vorteilhafte Ausgestaltungen der Erfindung sind Gegenstand der Unteransprüche.

Der Kerngedanke der vorliegenden Erfindung besteht demzufolge nicht nur darin, eine Sterilverpackung mit einer (inneren) ersten Sterilbarriere und einer (äußeren) zweiten Sterilbarriere bereitzustellen, die in einer entsprechenden Umverpackung untergebracht ist, wie dies im Stand der Technik vorgeschlagen wird, sondern darüber hinaus durch einen speziellen Aufbau der Umverpackung die Eigenschaften und Funktionen der jeweiligen äußeren zweiten Sterilbarriere und der Umverpackung miteinander zu kombinieren.

In anderen Worten ausgedrückt ist die Umverpackung der Sterilverpackung als Verbundverpackung, beispielsweise im Sinne eines Getränkekartons, konzipiert. Auf diese Weise können durch die Umverpackung mehrere Funktionen realisiert und darüber hinaus die äußere zweite Sterilbarriere in der Umverpackung ausgebildet werden. So wird zum einen die aseptische Präsentation des in der Sterilverpackung enthaltenen Medizinproduktes während einer Operation einfacher, und zum anderen das Verpackungsvolumen und Verpackungsmaterial reduziert.

So ist im Konkreten eine Sterilverpackung für sterile Medizinprodukte, insbesondere Implantate, vorgesehen, welche eine (innere) erste Sterilbarriere und eine äußere zur ersten Sterilbarriere getrennt und unabhängig ausgebildete zweite Sterilbarriere aufbaut und welche in einer Umverpackung aufgenommen ist. Gemäß der Erfindung besteht die Umverpackung aus einem Verbundmaterial, unter anderem aus einem Karton und zumindest einer Beschichtung oder Folie, und ist dafür ausgebildet und vorgesehen, die äußere zweite Sterilbarriere der Sterilverpackung zu bilden. Darüber hinaus kann die Sterilverpackung gemäß der vorliegenden Erfindung auch bei anderen Sterilprodukten zum Einsatz kommen.

Gemäß einem weiteren Aspekt der Erfindung ist es vorteilhaft, wenn die Umverpackung zusätzlich von Zellophan umgeben ist. Zellophan wird im Vergleich zu anderen Kunststoffen aus nachwachsenden statt fossilen Rohstoffen produziert und ist kompostierbar. Dadurch ist Zellophan generell umweltfreundlicher als herkömmliche für Verpackungen genutzte Folien. Obwohl Zellophan zum einen wasserdicht ist, hat es zum anderen den Vorteil, Wasser als Dampfmoleküle durchzulassen, sodass eine Ansammlung von Wasser innerhalb der Sterilverpackung vermieden werden kann. Neben seiner Transparenz verfügt es zudem über ausgezeichnete Druckleistungen und kann mit gewöhnlicher Tinte bedruckt werden, weshalb es als Verpackungsmaterial generell bzw. als zusätzlicher Überzug für Verpackungen gut geeignet ist.

Um eine robuste und den Anforderungen an Verpackungen von Medizinprodukten entsprechende äußere (zweite) Sterilbarriere gewährleisten zu können, besteht das Verbundmaterial der Umverpackung nach einem anderen Aspekt der Erfindung aus einem Mehrschichtverbund, beispielsweise im Sinne eines Getränkekartons, aus Karton und Kunststofffolie, insbesondere Polyethylenfolie. Dabei verleiht der Karton dem Verbundmaterial Form und Stabilität und bietet zudem eine glatte Oberfläche für das Bedrucken der Sterilverpackung. Die Polyethylenfolie schützt im Fall der Doppel-Sterilverpackung wiederum die innere als erste Sterilbarriere ausgebildete Verpackung und das sich darin befindliche Medizinprodukt vor Feuchtigkeit. Nach einem weiteren Aspekt der vorliegenden Erfindung kann das Verbundmaterial der Umverpackung auch aus einem Mehrschichtverbund aus Karton, Kunststofffolie, insbesondere Polyethylenfolie, und Aluminiumfolie bestehen. In diesem Fall dient die Polyethylenfolie neben dem Schutz vor Feuchtigkeit auch als Haftschicht zwischen dem Karton und der Aluminiumfolie. Die Aluminiumfolie hat die Aufgabe das Füllgut, d.h. das sich in der Umverpackung in der inneren als ersten Sterilbarriere ausgebildeten Verpackung befindliche Medizinprodukt, vor äußeren Einflüssen, wie zum Beispiel extremen Temperaturen, zu schützen. So kann die Sterilverpackung im Konkreten derart konzipiert sein, dass der Mehrschichtverbund von innen nach außen aus dem Karton, zwei Schichten Polyethylenfolie, einer Schicht Aluminiumfolie gefolgt von einer weiteren Schicht Polyethylenfolie, einer Schicht Papier und einer äußeren Schicht Polyethylenfolie besteht. Dabei schützt die äußerste Polyethylenfolie den Karton zusätzlich vor Durchnässung und erhöht die Barriereeigenschaften des Mehrschichtverbunds. Alternativ kann der Mehrschichtverbund auch über einen anderen Aufbau bzw. eine andere Anordnung verfügen und je nach Funktion variabel gestaltet werden.

Eine Integration von ggf. auch gasdurchlässigen Bereichen im Mehrschichtverbund, um neben eines gängigen Sterilisationsverfahren wie der Gammasterilisation auch weitere gebräuchliche Verfahren wie bspw. die Sterilisation mit Ethylenoxid zu ermöglichen ist eine weitere Ausprägungsform der vorliegenden Erfindung.

Um die Umverpackung öffnen und das darin aufbewahrte sterile Medizinprodukt, welches sich in der inneren als erste Sterilbarriere ausgebildeten Verpackung befindet, entnehmen zu können, weist die äußere als zweite Sterilbarriere der Sterilverpackung ausgebildete Umverpackung gemäß einem Aspekt der Erfindung eine Sollbruchstelle auf. Nach einem weiteren Aspekt der Erfindung befindet sich die Sollbruchstelle an einem oberen Drittel der äußeren als zweite Sterilbarriere der Sterilverpackung ausgebildeten Umverpackung. Dabei ist die Sollbruchstellte nach einem wiederum anderen Aspekt der Erfindung perforiert. So wird gewährleistet, dass die "unsterile OP-Schwester" die Umverpackung vergleichsweise einfach und ohne auftretende Komplikationen öffnen bzw. abknicken kann. Ein derartiger Öffnungsmechanismus der Umverpackung reduziert dabei zeitgleich das Risiko, dass das sterile Medizinprodukt, welches sich in der inneren als erste Sterilbarriere ausgebildeten Verpackung befindet, zu Boden fällt und beschädigt wird und aufgrund dessen nicht mehr benutzt werden kann.

Damit durch die perforierte Sollbruchstelle die intakte Doppel-Sterilbarriere nicht gefährdet wird, darf die Perforation nicht alle Schichten umfassen. Gemäß einem Aspekt der Erfindung sind daher mit Ausnahme der innersten Schicht des Mehrschichtverbunds des Verbundmaterials der Umverpackung alle Schichten des Mehrschichtverbunds perforiert. Alternativ können aber auch weniger oder nur bestimmte Schichten des Mehrschichtverbunds perforiert sein. In anderen Worten können mehr Schichten des Mehrschichtverbunds als nur die innerste Schicht frei von Perforation sein.

Nach einem anderen Aspekt der Erfindung kann die Sollbruchstelle zusätzlich mit einer Aufreißlasche/Abziehlasche versehen sein. Eine zusätzlich zu der Sollbruchstelle in der Umverpackung eingearbeitete Aufreißlasche/Abziehlasche stellt eine effiziente Alternative für ein einfacheres und unkomplizierteres Öffnen der Sterilverpackung dar.

Schließlich steht die innere als erste Sterilbarriere ausgebildete Verpackung, in welcher mindestens ein steriles Medizinprodukt untergebracht ist, gemäß einem weiteren Aspekt der Erfindung bei geöffneter/abgeknickter äußerer als zweite Sterilbarriere ausgebildeter Umverpackung über. Das heißt, ein oberer Teil der inneren als erste Sterilbarriere ausgebildeten Verpackung ragt über die geöffnete/abgeknickte äußere als zweite Sterilbarriere ausgebildete Umverpackung heraus. So kann die innere als erste Sterilbarriere ausgebildete Verpackung von der "sterilen OP-Schwester" einfach und problemlos entnommen werden, ohne das ein umständliches Hineingreifen in die Umverpackung nötig ist.

An dieser Stelle sei ausdrücklich darauf hingewiesen, dass die vorstehenden Aspekte einzeln sowie in beliebiger Kombination miteinander die gestellte Aufgabe lösen können und daher einzeln oder in beliebiger Kombination im Rahmen dieser Anmeldung beanspruchbar sein sollen.

### Kurzbeschreibung der Figuren

Die vorliegende Erfindung wird im Folgenden anhand von bevorzugten Ausführungsbeispielen unter Bezugnahme der beigefügten Zeichnungen näher beschrieben. Es zeigen:
Fig. 1 eine Darstellung zur Veranschaulichung einer Sterilverpackung für sterile Medizinprodukte gemäß einem ersten Ausführungsbeispiel der vorliegenden Offenbarung;
Fig. 2 eine Draufsicht einer geöffneten Sterilverpackung für sterile Medizinprodukte gemäß dem ersten Ausführungsbeispiel der vorliegenden Offenbarung;
Fig. 3 eine Vorderansicht der geöffneten Sterilverpackung für sterile Medizinprodukte gemäß dem ersten Ausführungsbeispiel der vorliegenden Offenbarung;
Fig. 4 eine Darstellung zur Veranschaulichung einer Sterilverpackung für sterile Medizinprodukte gemäß einem zweiten Ausführungsbeispiel der vorliegenden Offenbarung; und
Fig. 5 eine Darstellung eines typischen Aufbaus einer Umverpackung der Sterilverpackung gemäß der vorliegenden Offenbarung.

### Beschreibung der Ausführungsbeispiele

Nachstehend werden Ausführungsbeispiele der vorliegenden Offenbarung auf der Basis der zugehörigen Figuren beschrieben. Gleiche oder funktional äquivalente Merkmale sind in den einzelnen Figuren mit denselben Bezugszeichen versehen.

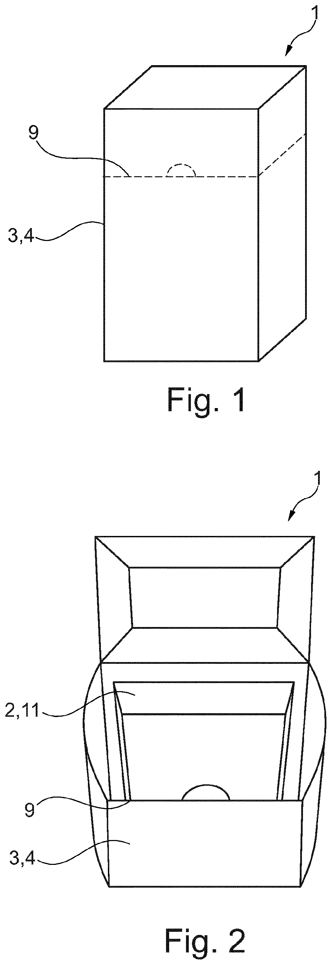

Fig. 1 zeigt eine noch ungeöffnete Sterilverpackung 1 für sterile Medizinprodukte, insbesondere für Implantate, welche eine in Fig. 1 nicht dargestellte innere erste Sterilbarriere 2 und eine äußere zur ersten Sterilbarriere 2 getrennt und unabhängig ausgebildete zweite Sterilbarriere 3 aufbaut und welche in einer Umverpackung 4 aufgenommen ist. Die Umverpackung 4 besteht dabei aus einem in Fig. 1 nicht näher dargestellten Verbundmaterial, unter anderem aus einem Karton 5 und zumindest einer Beschichtung oder Folie, und ist dafür ausgebildet und vorgesehen, die zweite Sterilbarriere 3 der Sterilverpackung 1 zu bilden.

Des Weiteren weist die äußere als zweite Sterilbarriere 3 der Sterilverpackung 1 ausgebildete Umverpackung 4 eine Sollbruchstelle 9 auf, welche zum Öffnen der Umverpackung 4 dient. Da die Umverpackung 4 in Fig. 1 noch ungeöffnet/verschlossen ist, ist sowohl die Sollbruchstelle 9, als auch die äußere zweite Sterilbarriere 3 noch intakt. Die Sollbruchstelle 9 ist an einem oberen Drittel der als zweite Sterilbarriere 3 der Sterilverpackung 1 ausgebildeten Umverpackung 4 angeordnet, um eine Entnahme einer inneren als erste Sterilbarriere 2 ausgebildeten Verpackung 11, die sich in der als zweite Sterilbarriere 3 ausgebildeten Umverpackung 4 befindet, zu erleichtern. Gemäß dem ersten Ausführungsbeispiel in Fig. 1 wird die Sollbruchstelle 9 durch Perforation der Umverpackung 4 erreicht. Um bei einer Vorperforation die äußere zweite Sterilbarriere 3 nicht zu gefährden und die Sterilität des in der Umverpackung 4 befindlichen Medizinproduktes zu gewährleisten, darf die Vorperforation nicht alle Schichten umfassen. Das heißt, zumindest die innerste Schicht des Verbundmaterials ist nicht perforiert. Daher sind gemäß der vorliegenden Erfindung mit Ausnahme der innersten Schicht des Mehrschichtverbunds des Verbundmaterials der Umverpackung 4 alle Schichten des Mehrschichtverbunds perforiert. Alternativ können aber auch weniger oder nur bestimmte Schichten des Mehrschichtverbunds perforiert sein. In anderen Worten können mehr Schichten des Mehrschichtverbunds als nur die innerste Schicht frei von Perforation sein.

Zudem kann die Sterilverpackung 1 von Zellophan, welches in Fig. 1 nicht weiter dargestellt ist, umgeben/ummantelt sein, welches die Sterilverpackung 1 durch seine wasserabweisenden Eigenschaften zusätzlich vor Feuchtigkeit schützt und eine Ansammlung von Wasser innerhalb der Sterilverpackung 1 vermeidet. In der Praxis entfernt eine "unsterile OP-Schwester" das ggf. die Umverpackung 4 umgebende Zellophan und öffnet die äußere als zweite Sterilbarriere 3 ausgebildete Umverpackung 4 durch Abknicken/Aufbrechen der Umverpackung 4 an der Sollbruchstelle 9. Dadurch wird zeitgleich auch die äußere zweite Sterilbarriere 3 durchbrochen.

In Fig. 2 ist die Sterilverpackung 1 gemäß dem ersten Ausführungsbeispiel der vorliegenden Offenbarung aus Fig. 1 in geöffnetem Zustand von oben gezeigt. Durch das Öffnen bzw. Abknicken der äußeren als zweite Sterilbarriere 3 ausgebildeten Umverpackung 4 an der Sollbruchstelle 9 wird die intakte innere als erste Sterilbarriere 2 ausgebildete Verpackung 11, beispielsweise in Form eines sterilen Blisters, Peelbeutel o.ä., freigelegt und kann von der "sterilen OP-Schwester" entnommen werden.

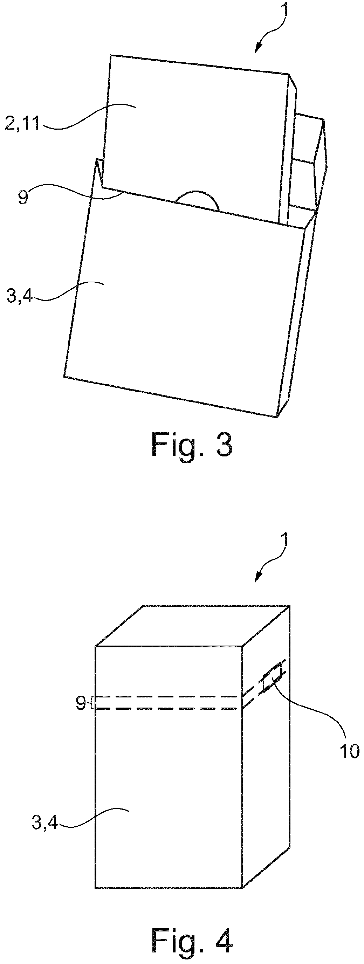

Fig. 3 zeigt die geöffnete Sterilverpackung 1 gemäß dem ersten Ausführungsbeispiel der vorliegenden Offenbarung aus Fig. 2 von vorn. Aus dieser Perspektive wird ersichtlich inwiefern die Anordnung der Sollbruchstelle 9 an einem oberen Drittel der äußeren als zweite Sterilbarriere 3 ausgebildeten Umverpackung 4 vorteilhaft für das Entnehmen der inneren als erste Sterilbarriere 2 ausgebildeten

Verpackung 11 ist. Um das Verpackungsvolumen und dadurch das Anfallen von Verpackungsmüll zu reduzieren, sind die innere als erste Sterilbarriere 2 ausgebildete Verpackung 11 und die äußere als zweite Sterilbarriere 3 ausgebildete Umverpackung 4 in ihren Größenordnungen/Dimensionen in Abhängigkeit von dem jeweils verpackten Medizinprodukt aneinander angepasst. Das heißt, eher kleine Medizinprodukte, die in einer für ihre Größe angemessenen inneren als erste Sterilbarriere 2 ausgebildeten Verpackung 11 untergebracht sind, werden nicht in einer unverhältnismäßig großen äußeren als zweite Sterilbarriere 3 ausgebildeten Umverpackung 4 untergebracht. Dies gewährleistet, dass die innere als erste Sterilbarriere 2 ausgebildete Verpackung 11 bei geöffneter als zweite Sterilbarriere 3 ausgebildeter Umverpackung 4 übersteht bzw. über die geöffnete als zweite Sterilbarriere 2 ausgebildete Umverpackung 4 herausragt. Die innere als erste Sterilbarriere 2 ausgebildete Verpackung 11 kann durch die "sterile OP-Schwester" nun aufgrund des vorliegenden Überstandes einfach entnommen und das sterile Medizinprodukt dem behandelnden Arzt nach Öffnen der inneren als erste Sterilbarriere 2 ausgebildeten Verpackung 11 angereicht werden.

Fig. 4 zeigt ein zweites erfindungsgemäßes Ausführungsbeispiel der Sterilverpackung 1 für Medizinprodukte. Gleiche Elemente und/oder Komponenten des ersten Ausführungsbeispiels aus Fig. 1 bis 3 sind mit denselben Bezugszeichen versehen, sodass im Folgenden nur auf Unterschiede zwischen dem ersten und zweiten Ausführungsbeispiel eingegangen wird. Bei diesem Ausführungsbeispiel ist die Sterilverpackung 1 an der Sollbruchstelle 9 zusätzlich mit einer Aufreißlasche/Abziehlasche 10 versehen. Die Aufreißlasche/Abziehlasche 10 dient dabei in Ergänzung zu der Sollbruchstelle 9 als weitere Möglichkeit die Sterilverpackung 1 einfacher zu Öffnen als dies bisher im Stand der Technik möglich ist. Durch Ziehen an der Aufreißlasche/Abziehlasche 10 kann die äußere als zweite Sterilbarriere 3 ausgebildete Umverpackung 4 an der Sollbruchstelle 9 ohne Komplikationen und ohne verhältnismäßig viel Kraft aufgerissen/geöffnet werden.

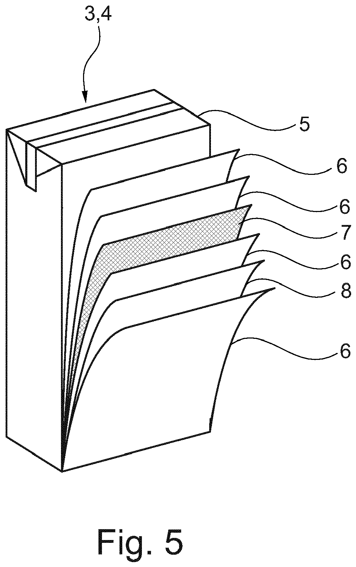

In Fig. 5 ist ein typischer Aufbau einer Umverpackung 4 der Sterilverpackung 1 gemäß der vorliegenden Offenbarung dargestellt. Die Umverpackung 4 ist gemäß der vorliegenden Erfindung als Verbundverpackung, beispielsweise nach Art eines Getränkekartons, bestehend aus sogenannten Verbundmaterialien konzipiert. Das Verbundmaterial der Umverpackung 4 besteht dabei in Fig. 5 aus einem Mehrschichtverbund aus Karton 8, Kunststoffolie 6, insbesondere Polyethylenfolie 6, und Aluminiumfolie 7. Jedoch kann das Verbundmaterial der Umverpackung 4 alternativ auch lediglich aus einem Mehrschichtverbund aus Karton 5 und Kunststoffolie 6, insbesondere Polyethylenfolie 6, bestehen. Die einzelnen Materialkomponenten erfüllen in diesem Zusammenhang unterschiedliche Funktionen. So verleiht der Karton 8 dem Verbundmaterial Form und Stabilität und bietet des Weiteren eine glatte Oberfläche für das Bedrucken der Sterilverpackung 1. Die Kunststofffolie bzw. Polyethylenfolie 6 schützt wiederum die innere als erste Sterilbarriere 2 ausgebildete Verpackung 11 und das sich darin befindende Medizinprodukt vor Feuchtigkeit und dient als Haftschicht zwischen dem Karton 5 und der Aluminiumfolie 7. Die Aluminiumfolie 7 schützt das sich in der Umverpackung 4 in der inneren als erste Sterilbarriere 2 ausgebildeten Verpackung 11 befindliche Medizinprodukt wiederum vor äußeren Einflüssen, wie beispielsweise extremen Temperaturen. Für eine effiziente Kombination der einzelnen Funktionen der Materialkomponenten kann der Mehrschichtverbund von innen nach außen aus zwei Schichten Polyethylenfolie 6, einer Schicht Aluminiumfolie 7 gefolgt von einer weiteren Schicht Polyethylenfolie 6, einer Schicht Papier bzw. Karton 8 und einer äußeren Schicht Polyethylenfolie 6 bestehen. Alternativ kann der Mehrschichtverbund aber auch anders aufgebaut sein und je nach Funktion variabel gestaltet werden.

Zusammenfassend betrifft die Erfindung folglich eine Sterilverpackung 1 für sterile Medizinprodukte, insbesondere Implantate, welche eine innere erste Sterilbarriere 2 und eine äußere zur ersten Sterilbarriere 2 getrennt und unabhängig ausgebildete zweite Sterilbarriere 3 aufbaut und welche in einer Umverpackung 4 aufgenommen ist. Erfindungsgemäß besteht die Umverpackung 4 einer derartigen Sterilverpackung 1 aus einem Verbundmaterial, unter anderem aus einem Papier/Karton 8 und zumindest einer Beschichtung oder Folie, und ist dafür ausgebildet und vorgesehen, die zweite Sterilbarriere 3 der Sterilverpackung 1 zu bilden.

Die Erfindung betrifft zusammengefasst eine Sterilverpackung für sterile Medizinprodukte, insbesondere Implantate, welche als Mehrfach-Sterilverpackung zumindest eine innere erste Sterilbarriere und eine äußere zur ersten Sterilbarriere getrennt und unabhängig ausgebildete zweite Sterilbarriere aufbaut und welche in einer Umverpackung aufgenommen ist. Erfindungsgemäß besteht die Umverpackung einer derartigen Sterilverpackung aus einem Verbundmaterial, unter anderem aus einem Karton und zumindest einer Beschichtung oder Folie, und ist dafür ausgebildet und vorgesehen, die zweite Sterilbarriere der Sterilverpackung zu bilden.

### Bezugszeichenliste

- 1: Sterilverpackung
- 2: Innere erste Sterilbarriere
- 3: Äußere zweite Sterilbarriere
- 4: Umverpackung
- 5: Verbundverpackung
- 6: Kunststofffolie/Polyethylenfolie
- 7: Aluminiumfolie
- 8: Papier/Karton
- 9: Sollbruchstelle
- 10: Aufreißlasche/Abziehlasche
- 11: Innere Verpackung

## Patentansprüche

1. Sterilverpackung (1) für sterile Medizinprodukte, welche als Doppel-Sterilverpackung eine innere erste Sterilbarriere (2) und eine äußere zur ersten Sterilbarriere (2) getrennt und unabhängig ausgebildete zweite Sterilbarriere (3) aufbaut und welche in einer Umverpackung (4) aufgenommen ist, **dadurch gekennzeichnet, dass** die Umverpackung (4) aus einem Verbundmaterial, unter anderem aus einem Karton (8) und zumindest einer Beschichtung oder Folie, besteht und die Umverpackung (4) und insbesondere deren Beschichtung oder Folie im Verbundmaterial dafür ausgebildet und vorgesehen ist, die äußere zweite Sterilbarriere (3) der Sterilverpackung (1) zu bilden und die innere als erste Sterilbarriere (2) ausgebildete Verpackung (11), in welcher mindestens ein steriles Medizinprodukt untergebracht ist, bei geöffneter äußerer als zweite Sterilbarriere (3) ausgebildeter Umverpackung (4) übersteht.

2. Sterilverpackung (1) für sterile Medizinprodukte nach Anspruch 1, **dadurch gekennzeichnet, dass** ein Teil der Umverpackung gasdurchlässig gestaltet ist

3. Sterilverpackung (1) für sterile Medizinprodukte nach Anspruch 1 oder 2, **dadurch gekennzeichnet, dass** die Umverpackung (4) zusätzlich von Zellophan umgeben ist.

4. Sterilverpackung (1) für sterile Medizinprodukte nach Anspruch 1 oder 3, **dadurch gekennzeichnet, dass** das Verbundmaterial der Umverpackung (4) aus einem Mehrschichtverbund aus Karton (5) und Kunststofffolie (6) besteht.

5. Sterilverpackung (1) für sterile Medizinprodukte nach Anspruch 4, **dadurch gekennzeichnet, dass** das Verbundmaterial der Umverpackung (4) aus einem Mehrschichtverbund aus Karton (8), Kunststofffolie (6) und Aluminiumfolie (7) besteht.

6. Sterilverpackung (1) für sterile Medizinprodukte nach Anspruch 5, **dadurch gekennzeichnet, dass** der Mehrschichtverbund von innen nach außen aus zwei Schichten Polyethylenfolie (6), einer Schicht Aluminiumfolie (7) gefolgt von einer weiteren Schicht Polyethylenfolie (6), einer Schicht Papier/Karton (8) und einer äußeren Schicht Polyethylenfolie (6) besteht.

7. Sterilverpackung (1) für sterile Medizinprodukte nach einem der Ansprüche 1 bis 6, **dadurch gekennzeichnet, dass** die äußere als zweite Sterilbarriere (3) der Sterilverpackung (1) ausgebildete Umverpackung (4) eine Sollbruchstelle (9) aufweist.

8. Sterilverpackung (1) für sterile Medizinprodukte nach Anspruch 7, **dadurch gekennzeichnet, dass** sich die Sollbruchstelle (9) an einem oberen Drittel der äußeren als zweite Sterilbarriere (3) der Sterilverpackung (1) ausgebildeten Umverpackung (4) befindet.

9. Sterilverpackung (1) für sterile Medizinprodukte nach Anspruch 7 oder 8, **dadurch gekennzeichnet, dass** die Sollbruchstelle (9) perforiert ist.

10. Sterilverpackung (1) für sterile Medizinprodukte nach einem der Ansprüche 1 bis 9, **dadurch gekennzeichnet, dass** zumindest eine Schicht, bevorzugt die innerste Schicht, des Mehrschichtverbunds des Verbundmaterials der Umverpackung (4) nicht perforiert ist.

11. Sterilverpackung (1) für sterile Medizinprodukte nach einem der Ansprüche 1 bis 10, **dadurch gekennzeichnet, dass** mit Ausnahme der innersten Schicht des Mehrschichtverbunds des Verbundmaterials der Umverpackung (4) alle Schichten des Mehrschichtverbunds perforiert sind.

12. Sterilverpackung (1) für sterile Medizinprodukte nach Anspruch 7, **dadurch gekennzeichnet, dass** die Sollbruchstelle (9) zusätzlich mit einer Aufreißlasche/Abziehlasche (10) versehen ist.

## Claims

1. Sterile packaging (1) for sterile medical devices, which as a double sterile packaging builds up an inner first sterile barrier (2) and an outer second sterile barrier (3) formed separately from and independently of the first sterile barrier (2), and which is accommodated in an outer packaging (4), **characterized in that** the outer packaging (4) consists of a composite material, inter alia of cardboard (8) and at least one coating or film, and the outer packaging (4) and in particular its coating or film in the composite material is designed and intended to form an outer second sterile barrier (3) of the sterile packaging (1) and the inner packaging (11) formed as a first sterile barrier (2), in which at least one sterile medical device is accommodated, protrudes when the outer packaging (4) formed as a second sterile barrier (3) is opened.

2. Sterile packaging (1) for sterile medical devices according to claim 1 **characterized in that** a part of the outer packaging is designed to be gas-permeable.

3. Sterile packaging (1) for sterile medical devices according to claim 1 or 2, **characterized in that** the outer packaging (4) is additionally surrounded by cellophane.

4. Sterile packaging (1) for sterile medical devices according to claim 1 or 3, **characterized in that** the composite material of the outer packaging (4) consists of a multilayer composite of cardboard (5) and plastic foil (6).

5. Sterile packaging (1) for sterile medical devices according to claim 4 **characterized in that** the composite material of the outer packaging (4) consists of a multilayer composite of cardboard (8), plastic foil (6) and aluminum foil (7).

6. Sterile packaging (1) for sterile medical devices according to claim 5, **characterized in that** the multilayer composite from the inside to the outside consists of two layers of polyethylene film (6), a layer of aluminum foil (7) followed by another layer of polyethylene film (6), a layer of paper/cardboard (8) and an outer layer of polyethylene film (6).

7. Sterile packaging (1) for sterile medical devices according to any of claims 1 to 6, **characterized in that** the outer outer packaging (4) formed as a second sterile barrier (3) of the sterile packaging (1) has a predetermined breaking point (9).

8. Sterile packaging (1) for sterile medical devices according to claim 7, **characterized in that** the predetermined breaking point (9) is located on an upper third of the outer packaging (4) formed as a second sterile barrier (3) of the sterile packaging (1).

9. Sterile packaging (1) for sterile medical devices according to claim 7 or 8, **characterized in that** the predetermined breaking point (9) is perforated.

10. Sterile packaging (1) for sterile medical devices according to any of claims 1 to 9, **characterized in that** at least one layer, preferably the innermost layer, of the multilayer composite of the composite material of the outer packaging (4) is not perforated.

11. Sterile packaging (1) for sterile medical devices according to any of claims 1 to 10, **characterized in that**, with the exception of the innermost layer of the multilayer composite of the composite material of the outer packaging (4), all layers of the multilayer composite are perforated.

12. Sterile packaging (1) for sterile medical devices according to claim 7, **characterized in that** the predetermined breaking point (9) is additionally provided with a tear-off lug/pull-off lug (10).

## Revendications

1. Emballage stérile (1) pour produits médicaux stériles, qui constitue en tant que double emballage stérile une première barrière stérile interne (2) et une deuxième barrière stérile externe (3) conçue séparément et indépendamment de la première barrière stérile (2) et qui est reçu dans un emballage extérieur (4), **caractérisé en ce que** l'emballage extérieur (4) se compose d'un matériau composite, entre autres d'un carton (8) et au moins d'un revêtement ou d'un film, et l'emballage extérieur (4), et en particulier son revêtement ou film en matériau composite, est conçu et prévu pour former la deuxième barrière stérile externe (3) de l'emballage stérile (1) et l'emballage intérieur (11) conçu sous forme de première barrière stérile (2), dans lequel au moins un produit médical est abrité, reste en cas d'emballage extérieur (4) ouvert, conçu sous forme de deuxième barrière stérile (3).

2. Emballage stérile (1) pour des produits médicaux stériles selon la revendication 1, **caractérisé en ce qu'**une partie de l'emballage extérieur est conçu sous forme perméable au gaz.

3. Emballage stérile (1) pour des produits médicaux stériles selon la revendication 1 ou 2, **caractérisé en ce que** l'emballage extérieur (4) est entouré en plus de cellophane.

4. Emballage stérile (1) pour des produits médicaux stériles selon la revendication 1 ou 3, **caractérisé en ce que** le matériau composite de l'emballage extérieur (4) se compose d'un composite multicouches en carton (5) et en film plastique (6) .

5. Emballage stérile (1) pour des produits médicaux stériles selon la revendication 4, **caractérisé en ce que** le matériau composite de l'emballage extérieur (4) se compose d'un composite multicouches en carton (8), en film plastique (6) et en film d'aluminium (7).

6. Emballage stérile (1) pour des produits médicaux stériles selon la revendication 5, **caractérisé en ce que** le matériau composite multicouches se compose, de l'intérieur vers l'extérieur, de deux couches de film de polyéthylène (6), d'une couche de film d'aluminium (7), suivies d'une autre couche de film de polyéthylène (6), d'une couche de papier/carton (8) et d'une couche extérieure de film de polyéthylène (6).

7. Emballage stérile (1) pour des produits médicaux stériles selon l'une des revendications 1 à 6, **caractérisé en ce que** l'emballage extérieur (4) externe conçu sous la forme de deuxième barrière stérile (3) de l'emballage stérile (1) présente un point de rupture défini (9).

8. Emballage stérile (1) pour des produits médicaux stériles selon la revendication 7, **caractérisé en ce que** le point de rupture défini (9) se trouve à un tiers supérieur de l'emballage extérieur (4) externe conçu sous forme de deuxième barrière stérile (3) de l'emballage stérile (1).

9. Emballage stérile (1) pour des produits médicaux stériles selon la revendication 7 ou 8, **caractérisé en ce que** le point de rupture défini (9) est perforé.

10. Emballage stérile (1) pour des produits médicaux stériles selon l'une des revendications 1 à 9, **caractérisé en ce qu'**au moins une couche, de préférence la couche la plus interne, du composite multicouches du matériau composite de l'emballage extérieur (4) n'est pas perforée.

11. Emballage stérile (1) pour des produits médicaux stériles selon l'une des revendications 1 à 10, **caractérisé en ce que**, à l'exception de la couche la plus interne du composite multicouches du matériau composite de l'emballage extérieur (4), toutes les couches du composite multicouches sont perforées.

12. Emballage stérile (1) pour des produits médicaux stériles selon la revendication 7, **caractérisé en ce que** le point de rupture défini (9) est doté en plus d'une languette de déchirure/d'arrachage (10).
